Europäisches Patentamt

European Patent Office

Office européen des brevets

(11) Veröffentlichungsnummer: **0 343 592 B1**

(12) **EUROPÄISCHE PATENTSCHRIFT**

(45) Veröffentlichungstag der Patentschrift: **22.09.93**

(51) Int. Cl.5: **G01N 33/84**

(21) Anmeldenummer: **89109242.1**

(22) Anmeldetag: **23.05.89**

(54) **Verfahren und Reagenz zur Bestimmung von Eisen.**

(30) Priorität: **26.05.88 DE 3817907**

237-240

(43) Veröffentlichungstag der Anmeldung:
**29.11.89 Patentblatt 89/48**

(45) Bekanntmachung des Hinweises auf die
Patenterteilung:
**22.09.93 Patentblatt 93/38**

(84) Benannte Vertragsstaaten:
**AT BE CH DE ES FR GB GR IT LI LU NL SE**

(56) Entgegenhaltungen:
**EP-A- 0 130 537**
**EP-A- 0 306 859**

**PATENT ABSTRACTS OF JAPAN, Band 7, Nr.
270 (P-240)[1415], 2. Dezember 1983; & JP-
A-58 148 964 (JIYUN OKUDA) 05-09-1983**

**BIOLOGICAL ABSTRACTS, Band 83, 1987,
Zusammenfassung Nr. 53572, Philadelphia,
PA, US; P. HOROWITZ et al.: "Low concentration of guanidinium chloride expose apolar
surfaces and cause differential perturbation
in catalytic intermediates of rhodanese", &&
J. BIOL. CHEM. 261(33): 15652-15658. 1986**

**Clinical Chemistry, vol.23,Nr.2, 1977, Seiten**

(73) Patentinhaber: **BOEHRINGER MANNHEIM
GMBH**

**D-68298 Mannheim(DE)**

(72) Erfinder: **Herrmann, Uwe, Dr. rer. nat.
Wettersteinstrasse 4
D-8139 Bernried(DE)**
Erfinder: **Ruhm, Jürgen
Im Westenfeld 47
D-4760 Werl(DE)**

(74) Vertreter: **Huber, Bernhard, Dipl.-Chem. et al
Patentanwälte H. Weickmann, Dr. K. Fincke
F.A. Weickmann, B. Huber Dr. H. Liska, Dr. J.
Prechtel Kopernikusstrasse 9 Postfach 86 08
20
D-81635 München (DE)**

EP 0 343 592 B1

**Beschreibung**

Die Erfindung betrifft ein Verfahren zur Bestimmung von Eisen in Körperflüssigkeiten durch Freisetzung des gebundenen Eisens, Reduktion zu $Fe^{2+}$, Zugabe eines für den Nachweis von Eisen geeigneten Farbsystems und Messung des Farbstoffkomplexes sowie eine Reagenzkombination, die auch ohne Zusatz von aufhellenden Tensidmischungen für die Messung von lipämischen Seren geeignet ist.

Eisen-Stoffwechselstörungen, insbesondere Eisenmangel und Eisenresorptionsstörungen sind vor allem in der weiblichen Bevölkerung weit verbreitet. Der Nachweis von Eisen in Körperflüssigkeiten, insbesondere im Serum gehört daher zu den Standardbestimmungen in der medizinischen Analytik. Eisen wird mit der Nahrung zugeführt und über die Schleimhaut des Darmes aufgenommen. An Transferrin in dreiwertigem Zustand gebunden wird es dann zum Knochenmark transportiert, wo es hauptsächlich in das Hämoglobin eingebaut wird. Wird zu wenig Eisen aufgenommen, so kommt es zu anämischen Erscheinungen.

Die Bestimmung des Eisens im Serum gehört zu den am häufigsten durchgeführten Spurenelementanalysen in der klinischen Diagnostik. Hierzu sind verschiedene Verfahren bekannt. So ist in Clin.Chem. 26, (1980) 327-331 ein Verfahren beschrieben, bei dem das an Transferrin in Form eines Carbonatkomplexes gebundene, dreiwertige Eisen in stark saurem Milieu freigesetzt wird. Nachteil dieses Verfahrens ist es, daß die stark sauren Reagentien ätzende und korrodierende Eigenschaften aufweisen. Um diesen Nachteil auszuräumen, ist es beispielsweise aus Clin.Chem. 23, (1977) 237-240 und aus Z.klin.Chem. 3 (1965) 96-99 bekannt, Protein denaturierende Agentien wie konzentriertes Guanidiniumchlorid oder anionische Detergentien zur Freisetzung des Eisens zu verwenden.

In den bekannten Ausführungsformen dieser Verfahren treten bei der Bestimmung von Eisen in trüben lipämischen Seren Fehlmessungen auf. Weder Guanidiniumchlorid noch die anionischen Detergentien besitzen eine ausreichende Aufklarungskraft, um eine vollständige Beseitigung der Trübung zu erreichen.

Zur Lösung dieses Problems wurde in EP 130 537 vorgeschlagen, eine Mischung aus nichtionischen und anionischen Detergentien zur Freisetzung des Eisens im schwach sauren Medium zu verwenden. Es wurde jedoch gefunden, daß bei Verwendung dieser Detergentien zwar rasch und vollständig eine Aufklarung von lipämischen Seren erfolgt, aber für Seren mit einem erhöhten Gehalt an Immunglobulin (Gammopathie-Seren) eine Eintrübung beobachtet wird, die das Meßergebnis verfälschen kann. Zudem ist die Wiederfindung bei stark hämolytischen Seren nicht völlig befriedigend.

Dazu kommt, daß bei den bisher bekannten Verfahren stets Detergenz zugegen sein muß, sei es als Denaturierungsmittel oder zur Vermeidung von Trübungen oder Verhinderung von Wechselwirkungen zwischen Farbstoff und Probe. Die Gegenwart von Detergenzien ist jedoch insbesondere bei der Anwendung des Bestimmungsverfahrens auf Analysenautomaten nachteilig, da Detergentien Schaumbildung verursachen und dadurch die Messung erheblich stören können.

Es bestand daher die Aufgabe, ein Verfahren und ein Reagenz zur Bestimmung von Eisen in Serum zu schaffen, bei dem einerseits keine aggressiven Bestandteile verwendet werden müssen und andererseits eine Störung durch lipämische Seren vermieden werden kann, ohne daß die Messung durch hämolytische Proben oder Seren mit erhöhtem Immunglobulingehalt gestört wird.

Aus Gründen der Automatisierbarkeit und Rationalisierung ist es ferner wünschenswert, daß ohne schaumbildende Detergentien gearbeitet werden kann und daß keine vorhergehende Enteiweißung der Probe erforderlich ist.

Diese Aufgabe wird erfindungsgemäß gelöst durch ein Verfahren zur Bestimmung von Eisen im Serum unter Freisetzung des gebundenen Eisens durch Zusatz eines Protein-Denaturierungsmittels, Reduktion des freigesetzten Eisens zu $Fe^{2+}$, Zugabe einer Farbreagenzlösung und photometrische Messung des gebildeten Farbkomplexes, das dadurch gekennzeichnet ist, daß man das Serum mit einer ersten Lösung eines Denaturierungsmittels in einer Konzentration von 1 bis 8 Mol/l versetzt, nach Zugabe der ersten Lösung eine Trübungsmessung vornimmt, danach eine zweite Lösung zusetzt, die das Farbreagenz und ebenfalls ein Denaturierungsmittel in einer Konzentration von 1 bis 8 Mol/l, enthält, und danach den Farbkomplex bestimmt und zur Ermittlung der Eisenmenge den Trübungsmeßwert vom Meßwert der Farbkomplexlösung subtrahiert, wobei die Konzentration des Denaturierungsmittels in der zweiten Lösung weniger als 5 % von der Konzentration in der ersten Lösung abweicht und als Denaturierungsmittel ein Salz mit Alkali-, Erdalkali-, Amen- oder Guanidinium-Ionen als Kationen und Halogen, Sulfaten, Perchlorat, Thiocyanat, Acylat oder Heparinat als Anionen oder Harnstoff verwendet werden.

Die Erfindung beruht auf der Feststellung, daß auch in Abwesenheit von Detergentien gearbeitet werden kann, wenn ein Zweischrittverfahren (1. Schritt: Ablösung des Eisens von den Bindeproteinen mit Denaturierungsmittel, 2. Schritt: Farbreaktion) angewendet wird, wobei eine Eintrübung beim Zusatz des Farbreagenzes vermieden werden kann, wenn dem Farbreagenz ebenfalls Denaturierungsmittel zugesetzt wird. Damit ist eine störungsfreie Messung auch von lipämischen Seren möglich.

Zur Ablösung des Eisens von Bindeproteinen, insbesondere von Ferritin, ist der Zusatz von verschiedenen Proteindenaturierungsreagentien bekannt. Neben Detergentien werden hierzu hohe Konzentrationen einer Vielzahl von Salzen eingesetzt, wie z.B. Guanidiniumchlorid oder Guanidinium-Acetat, Magnesiumsulfat (K. Lauber, Z.klin.Chem. 3, 1965, 96-99), Magnesiumchlorid (G. Brivio, La Ricerca Clin.Lab. 16, 1986, 523-532) und NaCl (EPA 0228060). In den bekannten Ausführungsformen, bei denen die Eisenbestimmung in einem zweistufigen Verfahren stattfindet, mit vorgeschalteter Denaturierungsstufe und nachgeschalteter Farbreaktion wird durch die Zugabe des Farbreagenzes die Ionenstärke des Denaturierungsreagenzes abgesenkt. Überraschenderweise stellte sich nun heraus, daß diese Modifikation der Ionenstärke für die Fehlmessung von lipämischen Seren im Eisentest verantwortlich ist.

Es wird angenommen, daß durch das Denaturierungsmittel erstens Eisen aus seinen Transportproteinen freigesetzt und zweitens, bei lipämischen Seren, ein definiertes Trübungsniveau eingestellt wird. Die Trübung wird daher vorzugsweise vor der Zugabe der Farbreagenzlösung gemessen und als Probenleerwert zum Abzug gebracht. Da erfindungsgemäß die Farbreagenzlösung eine nur wenig abweichende, bevorzugt dieselbe Konzentration des Denaturierungsmittels wie die Denaturierungslösung aufweist, bleibt während der Bildung des Farbkomplexes der Trübungshintergrund unbeeinflußt.

Zur Proteindenaturierung im Rahmen der Erfindung geeignet sind, neben den oben erwähnten Beispielen, generell hohe Konzentrationen der bei C. Tanford, Adv.Prot.Chem. 23, 121 ff., insbesondere S. 159, 173, 182-186, 1986, aufgeführten Protein-Denaturierungsmittel, wobei als Kationen Alkali- oder Erdalkalikationen, substituierte oder unsubstituierte Ammoniumverbindungen - oder andere organische Kationen, wie z.B. Guanidinium - und als Anionen Halogenide, Sulfate, Perchlorate, Thiocyanate oder andere anorganische oder auch organische Anionen, Acylat (wie z.B. Acetat) oder Heparinat in Frage kommen. Bevorzugte Reagenzien zur Denaturierung von Eisen-Bindeproteinen sind Harnstoff sowie Salze der Kationen Magnesium$^{2+}$ und Guanidinium, besonders bevorzugt die Salze des Guanidiniumkations wie z.B. -chlorid, -acetat, -thiocyanat oder -sulfat.

Die Konzentration des Denaturierungsmittels in beiden Lösungen des erfindungsgemäßen Eisentests muß relativ hoch sein und zwischen 1 und 8 Mol/l liegen. Beste Ergebnisse liefern Konzentrationen zwischen 4 und 6 Mol/l. Bei Konzentrationen von unter 1 Mol/l wird die Ablösung des Eisens von Transferrin zunehmend langsamer. Für das besonders bevorzugte Guanidiniumchlorid liegt der bevorzugte Konzentrationsbereich zwischen 1 bis 6 Mol/l, besonders bevorzugt zwischen 4 und 5 Mol/l.

Besonders vorteilhaft ist eine möglichst weitgehende Übereinstimmung der Konzentrationen des Denaturierungsmittels in beiden Lösungen. Ist nämlich die Konzentration im Farbreagenz geringer als im Vorinkubationsreagenz, kann es bei der Zugabe des Farbreagenzes zu lipämischen Serumproben zu einer Wiedereintrübung kommen, die einen höheren Eisengehalt der Probe vortäuscht. Im umgekehrten Fall, Denaturierungsmittel im Farbreagenz konzentrierter, kann eine nachträgliche weitere Aufklarung auftreten, wodurch das Eisensignal zu niedrig ist. Daher sollten vorzugsweise Vorinkubationsreagenz und Farbreagenz um weniger als 5 % in der Konzentration des Denaturierungsagenzes differieren.

Zur Durchführung des erfindungsgemäßen Verfahrens wird die Probelösung bevorzugt in einem schwach sauren Bereich, besonders bevorzugt im Bereich zwischen pH 4 und 6, abgepuffert. Als Puffersubstanzen sind Verbindungen geeignet, die einen pK-Wert von 4 bis 6 aufweisen und Eisen nicht komplexieren. Geeignet sind beispielsweise Acetat-, Phosphat-, Succinat- und Tris-Puffer.

Bevorzugt wird als Puffersubstanz Acetatpuffer eingesetzt. Der Puffer wird bevorzugt in einer Konzentration von 20 bis 500 mmol/l, besonders bevorzugt 50 bis 150 mMol/l verwendet.

Zur Bestimmung des Eisens wird dann in an sich bekannter Weise der Probelösung ein Reduktionsmittel wie z.B. Ascorbinsäure oder Dithionit zugesetzt, um das in dreiwertiger Form vorliegende freigesetzte Eisen in die zweiwertige Form zu reduzieren. Das Reduktionsmittel wird vorzugsweise der ersten Lösung zugesetzt. Weiterhin wird ein für den Nachweis von Eisen geeignetes Farbsystem zugegeben. Derartige Farbsysteme sind z.B. beschrieben in EP 228060, Clin.Biochem. 14 (1981) 311-315 und Clin.Chem. 23 (1979) 237-240. Besonders sind Komplexbildner vom Ferrointyp geeignet, die mit Eisen einen Farbstoff liefern, der photometrisch ausgewertet werden kann. Als geeignete Substanzen sind Bathophenanthrolin und 3'(2'-Pyridyl)-5,6-diphenyl-1,2,4-triazin-sulfonsäure-dinatriumsalz zu nennen. Die Farbstoffbildung erfolgt dabei proportional zum Eisengehalt der Probe und kann in an sich bekannter Weise photometrisch ausgewertet werden.

Ein weiterer Gegenstand der Erfindung ist eine Reagentienkombination zur Bestimmung von Eisen im Serum, gekennzeichnet durch ein erstes Reagens, enthaltend

20 bis 500 mMol/l Puffersubstanz, pH 4 bis 6

1 bis 8 Mol/l Denaturierungsmittel und

0,1 bis 100 mMol/l Reduktionsmittel

und getrennt davon ein zweites Reagens, enthaltend

20 bis 500 mMol/l Puffersubstanz, pH 4 bis 6

1 bis 8 Mol/l Denaturierungsmittel und

0,5 bis 50 mMol/l Farbstoff

in Form wäßriger Lösungen oder zu deren Herstellung geeigneter Trockengemische, oder vorzugsweise enthält Reagens 1

5 bis 50 mMol/l Reduktionsmittel

4 bis 6 Mol/l Denaturierungsmittel und

50 bis 150 mMol/l Puffer

und Reagens 2

4 bis 6 Mol/l Denaturierungsmittel

50 bis 150 mMol/l Puffer und

1 bis 20 mMol/l Farbstoff,

wobei als Denaturierungsmittel ein Salz mit Alkali-, Erdalkali-, Amin- oder Guanidinium- Ionen als Kationen und Halogen, Sulfaten Perchlorat, Thiocyanat, Acylat oder Heparinat als Anionen oder Harnstoff vorhanden sind.

Die folgenden Beispiele erläutern die Erfindung näher.

**Beispiel 1**

Zur Eisenbestimmung in Serum werden folgende Reagentien eingesetzt:

| Reagenz 1 | |
|---|---|
| - Guanidiniumhydrochlorid | 4,5 mol/l |
| - Acetatpuffer pH = 5,0 | 0,15 mol/l |
| - Ascorbinsäure | 0,023 mol/l |

| Reagenz 2 | |
|---|---|
| - Guanidiniumhydrochlorid | 4,5 mol/l |
| - Acetatpuffer pH = 5,5 | 0,02 mol/l |
| - Ferrozin$^R$ | 1,7 mmol/l |
| (3-(2-Pyridyl)-5-6-diphenyl-1,2,4-triazinsulfonsäuredinatriumsalz) | |

In eine Küvette wird zu einem Probevolumen von 40 $\mu$l ein Volumen von 700 $\mu$l des Denaturierungsreagenzes 1 pipettiert, 5 min bei T = 25°C inkubiert und bei 570 nm die Extinktion gemessen ($E_{1\ Probe}$). Danach werden 100 $\mu$l des Farbreagenzes $R_2$ zugesetzt. Nach weiteren 5 min wird das Farbsignal gemessen ($E_{2\ Probe}$). Ein Reagentienleerwert wird vor ($E_{1RL}$) und nach ($E_{2RL}$) Zugabe des Farbreagenzes gemessen, wobei statt der Probe 40 $\mu$l bidestilliertes Wasser eingesetzt werden. Das für die Eisenbestimmung resultierende Meßsignal ergibt sich demnach aus:

$$\Delta E = (E_{2\ Probe} - \left[\frac{740}{840} \cdot E_{1Probe}\right]) - (E_{2\ RL} - \left[\frac{740}{840} \cdot E_{1\ RL}\right])$$

Zur Kalibration der Eisenbestimmung wird ein Kontrollserum mit bekanntem Eisengehalt eingesetzt.

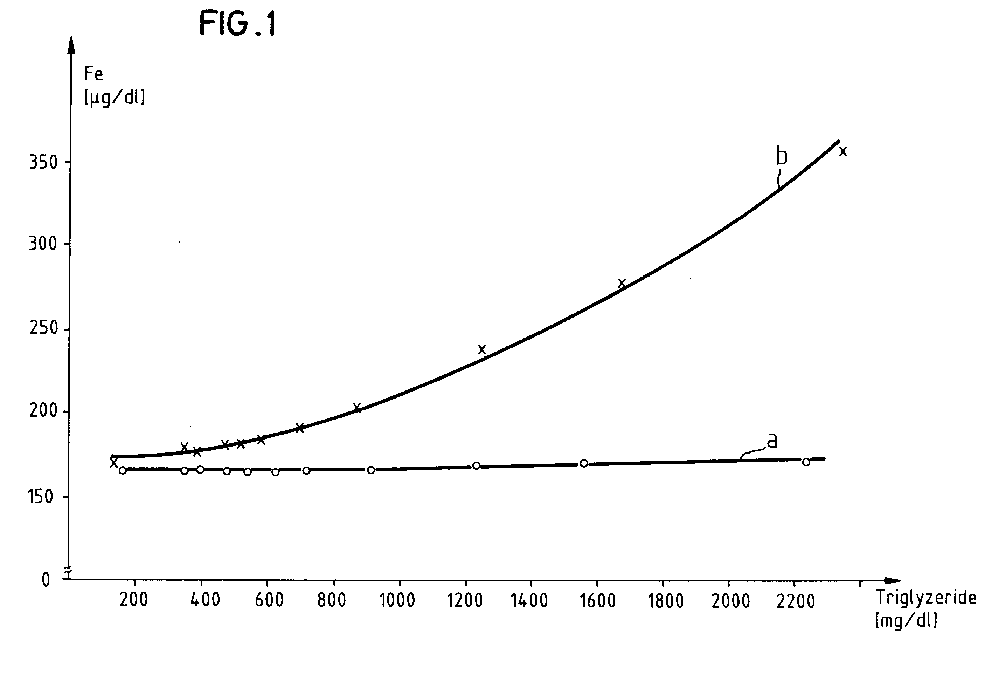

Als Proben wurden Mischungen aus einem Kontrollserum mit bekanntem Eisengehalt von 116 $\mu$g/dl und einer stark trüben Fettemulsion (intralipid$^R$), Pfrimmer & Co., Erlangen) hergestellt. Es wird zunächst eine wäßrige Vorverdünnungsreihe dieser Emulsion im Bereich von 1 Teil intralipid + 10 Teile Wasser bis 1 Teil + 0,5 Teile Wasser angesetzt, wobei eine zunehmende Trübung auftritt und ein steigender TG-Gehalt gemessen wird (Triglyzeridtest, gemessen an Hitachi 704). Aus dieser Vorverdünnungsreihe wird jeweils ein Teil entnommen und mit 19 Teilen Kontrollserum versetzt. Die Ergebnisse der Eisenwiederfindung sind in Figur 1 gegen den Triglyzerid-Gehalt aufgetragen. Kurvenzug a zeigt die Trübungsunabhängigkeit der Eisenwiederfindung mit der oben beschriebenen Rezeptur, während Kurve b die Ergebnisse mit analoger Rezeptur bei Fortlassung des Guanidiniumchlorids aus $R_2$ darstellt.

**Beispiel 2**

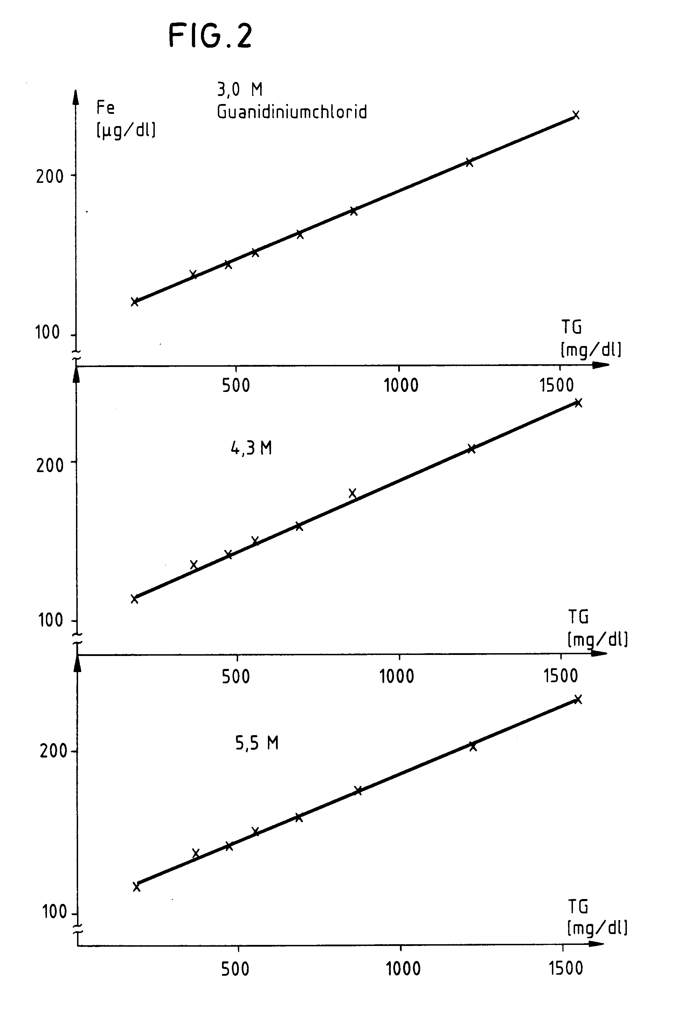

Bei einer zu Beispiel 1 analogen Meßweise wurde die Konzentration des Denaturierungsagenz Guanidiniumhydrochlorid in $R_1$ in weiten Bereichen variiert, während dem Farbreagenz kein Denaturierungsagenz zugesetzt war. Aus Figur 2 ist ersichtlich, daß die Fehlmessung nicht von der Konzentration des Denaturierungsagenz abhängt, sondern demnach, wie in Beispiel 1 dargestellt, vom Zusatz in Reagenz 2.

**Beispiel 3**

Bei einer zu Beispiel 1 analogen Meßweise wurde im Reagenz 2 der Farbstoff fortgelassen und statt 4,5 Mol/l Guanidiniumhydrochlorid verschiedene andere Agentien eingesetzt, die als Denaturierungsagentien bekannt sind. Gemessen wurde die Trübungsänderung bei 570 nm, wegen des fehlenden Farbreagenzes ohne Überlagerung durch das Eisensignal, an einer intralipid-Probe, die der höchsten Triclycerid-Konzentration in Beispiel 1 entspricht. In Tabelle 1 ist die relative Trübungsänderung bei Zugabe des Reagenzes 2 aufgeführt:

Tabelle 1

| Relative Trübungsänderung bei 570 nm für verschiedene Zusätze in Reagenz 2 | |
|---|---|
| Zusatz: | $\Delta E_{570}$ [%] (Trübung) |
| - | 32,1 |
| 4,5 Mol/l Guanidiniumchlorid | 2,6 |
| 3,4 Mol/l Natriumchlorid | 20,4 |
| 5 Mol/l Harnstoff | 12,9 |

Es zeigt sich, daß durch Zusatz von verschiedenen Denaturierungsmitteln zu Reagenz 2 die relative Trübungsänderung stark vermindert werden kann.

**Patentansprüche**

1. Verfahren zur Bestimmung von Eisen im Serum unter Freisetzung des gebundenen Eisens durch Zusatz eines Protein-Denaturierungsmittels, Reduktion des freigesetzten Eisens zu $Fe^{2+}$, Zugabe einer Farbreagenzlösung und photometrische Messung des gebildeten Farbkomplexes, **dadurch gekennzeichnet,** daß man das Serum mit einer ersten Lösung eines Denaturierungsmittels in einer Konzentration von 1 bis 8 Mol/l versetzt, nach Zugabe der ersten Lösung eine Trübungsmessung vornimmt, danach eine zweite Lösung zusetzt, die das Farbreagenz und ebenfalls ein Denaturierungsmittel in einer Konzentration von 1 bis 8 Mol/l, enthält und danach den Farbkomplex bestimmt und zur Ermittlung der Eisenmenge den Trübungsmeßwert vom Meßwert der Farbkomplexlösung subtrahiert, wobei die Konzentration des Denaturierungsmittels in der zweiten Lösung weniger als 5 % von der Konzentration in der ersten Lösung abweicht und als Denaturierungsmittel ein Salz mit Alkali-, Erdalkali-, Amin- oder Guanidinium-Ionen als Kationen und Halogen, Sulfaten, Perchlorat, Thiocyanat, Acylat oder Heparinat als Anionen oder Harnstoff verwendet werden.

2. Verfahren nach Anspruch 1, **dadurch gekennzeichnet,** daß man als Denaturierungsmittel ein Guanidiniumsalz verwendet.

3. Verfahren nach Anspruch 2, **dadurch gekennzeichnet,** daß Guanidinhydrochlorid in einer Konzentration von 4 bis 5 Mol/l verwendet wird.

4. Verfahren nach einem der vorhergehenden Ansprüche, **dadurch gekennzeichnet,** daß man zur Reduktion des Eisens Ascorbinsäure zusetzt.

**5.** Verfahren nach einem der vorhergehenden Ansprüche,
**dadurch gekennzeichnet,**
daß man als Farbreagenz Bathophenanthrolin oder 3' (2'-Pyridyl)-5,6 -diphenyl-1,2,4-triazin-sulfonsäure-dinatriumsalz zusetzt.

**6.** Reagentienkombination zur Bestimmung von Eisen im Serum,
**gekennzeichnet durch**
ein erstes Reagenz, enthaltend
20 bis 500 mMol/l Puffersubstanz, pH 4 bis 6
1 bis 8 Mol/l Denaturierungsmittel und
0,1 bis 100 mMol/l Reduktionsmittel
und getrennt davon ein zweites Reagenz, enthaltend
20 bis 500 mMol/l Puffersubstanz, pH 4 bis 6
1 bis 8 Mol/l Denaturierungsmittel und
0,5 bis 50 mMol/l Farbstoff
in Form wäßriger Lösungen oder zu deren Herstellung geeigneter Trockengemische, wobei als Denaturierungsmittel ein Salz mit Alkali-, Erdalkali-, Amin- oder Guanidinium-Ionen als Kationen und Halogen, Sulfaten, Perchlorat, Thiocyanat, Acylat oder Heparinat als Anionen oder Harnstoff vorhanden ist.

**Claims**

**1.** Method for the determination of iron in serum by addition of a protein-denaturing agent to release bound iron, reduction of the released iron to $Fe^{2+}$, addition of a colour reagent solution and photometric measurement of the colour complex which forms,
**wherein**
a first solution of a denaturing agent at a concentration of 1 to 8 mol/l is added to the serum, the turbidity is measured after addition of the first solution, afterwards a second solution is added containing the colour reagent and also a denaturing agent at a concentration of 1 to 8 mol/l and subsequently the colour complex is determined and the measured value of the turbidity is subtracted from the measured value of the colour complex solution in order to determine the amount of iron, wherein the concentration of the denaturing agent in the second solution differs by less than 5 % from the concentration in the first solution and a salt with alkaline, alkaline-earth, amine or guanidinium ions as cations and halogen, sulphates, perchlorate, thiocyanate, acylate or heparinate as anions or urea is used as the denaturing agent.

**2.** Method as claimed in claim 1,
**wherein**
a guanidinium salt is used as the denaturing agent.

**3.** Method as claimed in claim 2,
**wherein**
guanidine hydrochloride is used at a concentration of 4 to 5 mol/l.

**4.** Method as claimed in one of the previous claims,
**wherein**
ascorbic acid is added to reduce the iron.

**5.** Method as claimed in one of the previous claims,
**wherein**
bathophenanthroline or the disodium salt of 3'-(2'-pyridyl)-5,6-diphenyl-1,2,4-triazine-sulphonic acid is added as the colour reagent.

**6.** Reagent combination for the determination of iron in serum,
**wherein**
a first reagent is present containing
20 to 500 mmol/l buffering agent, pH 4 to 6
1 to 8 mol/l denaturing agent and

EP 0 343 592 B1

0.1 to 100 mmol/l reducing agent
and separate from this a second reagent is present containing
20 to 500 mmol/l buffering agent, pH 4 to 6
1 to 8 mol/l denaturing agent and
0.5 to 50 mmol/l dye
in the form of aqueous solutions or dry mixtures which are suitable for their production, wherein a salt with alkaline, alkaline-earth, amine or guanidinium ions as cations and halogen, sulphates, perchlorate, thiocyanate, acylate or heparinate as anions or urea is used as the denaturing agent.

**Revendications**

**1.** Procédé de détermination du fer dans le sérum avec libération du fer lié par addition d'un agent dénaturant les protéines, réduction du fer libéré en $Fe^{2+}$, addition d'une solution de réactif coloré et mesure photométrique du complexe coloré formé, caractérisé en ce que l'on additionne le sérum d'une première solution d'un agent dénaturant en une concentration de 1 à 8 mol/l, on réalise une première mesure de turbidité après l'addition de la première solution, puis on ajoute une seconde solution qui contient le réactif coloré et également un agent dénaturant en une concentration de 1 à 8 mol/l, puis on détermine le complexe coloré et, pour déterminer la quantité de fer, on soustrait la valeur de turbidité mesurée de la valeur mesurée pour la solution de complexe coloré, la concentration de l'agent dénaturant dans la seconde solution s'écartant de moins de 5% de la concentration dans la première solution et l'agent dénaturant utilisé étant un sel dont les cations sont des ions alcalins, alcalinoterreux, amines ou guanidinium et dont les anions sont des ions halogènes, sulfates, perchlorates, thiocyanates, acylates ou héparinates, ou l'urée.

**2.** Procédé selon la revendication 1, caractérisé en ce que l'on utilise un sel de guanidinium comme agent dénaturant.

**3.** Procédé selon la revendication 2, caractérisé en ce que l'on utilise le chlorhydrate de guanidine en une concentration de 4 à 5 mol/l.

**4.** Procédé selon l'une des revendications précédentes, caractérisé en ce que l'on ajoute de l'acide ascorbique pour la réduction du fer.

**5.** Procédé selon l'une des revendications précédentes, caractérisé en ce que l'on ajoute de la bathophénanthroline ou le sel disodique de l'acide 3'(2'-pyridyl)-5,6-diphényl-1,2,4-triazinesulfonique comme réactif coloré.

**6.** Combinaison de réactifs pour la détermination du fer dans le sérum, caractérisée par un premier réactif contenant
20 à 500 mmol/l de substance tampon, pH 4 à 6
1 à 8 mol/l d'agent dénaturant et
0,1 à 100 mmol/l d'agent réducteur
et, séparément de celui-ci, un second réactif contenant
20 à 500 mmol/l de substance tampon, pH 4 à 6
1 à 8 mol/l d'agent dénaturant et
0,5 à 50 mmol/l de colorant
sous forme de solutions aqueuses ou de mélanges secs convenant pour leur préparation, l'agent dénaturant étant présent sous forme d'un sel dont les cations sont des ions alcalins, alcalinoterreux, amines ou guanidinium et dont les anions sont des ions halogènes, sulfates, perchlorates, thiocyanates, acylates ou héparinates, ou sous forme d'urée.

7

FIG.1

Fe
[µg/dl]

350

300

250

200

150

0

200  400  600  800  1000  1200  1400  1600  1800  2000  2200

Triglyzeride
[mg/dl]

b

a

FIG.2